# EUROPÄISCHE PATENTSCHRIFT

(11) **EP 0 854 144 B1**
(45) Veröffentlichungstag und Bekanntmachung des Hinweises auf die Patenterteilung: **17.07.2002**
(21) Anmeldenummer: 98810003.8
(22) Anmeldetag: 06.01.1998
(51) Int. Cl.: C07D 311/78, C07C 51/54

(54) **Herstellung von Perylen-3,4-dicarbonsäureanhydriden**
Preparation of perylen-3,4-dicarboxylic acid anhydrides
Préparation d'acide perylene-3,4-dicarboxylique anhydrides

(30) Priorität: 14.01.1997 DE 19701009
(43) Veröffentlichungstag der Anmeldung: 22.07.1998
(73) Patentinhaber: Ciba Specialty Chemicals Holding Inc., 4057 Basel (CH)
(72) Erfinder: Feiler, Leonhard, 1762 Givisiez (CH)

(56) Entgegenhaltungen:
- EP-A- 0 205 980
- EP-A- 0 657 436
- WO-A-96/22331
- WO-A-98/31678

## Beschreibung

Die vorliegende Erfindung betrifft ein verbessertes Verfahren zur Herstellung von Perylen-3,4-dicarbonsäureanhydriden der allgemeinen Formel I 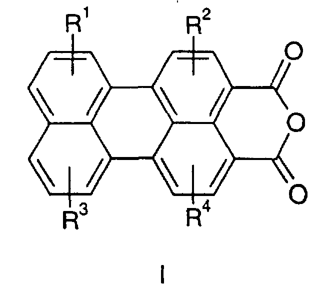 worin R¹, R², R³ und R⁴ unabhängig voneinander Wasserstoff, Halogen, C₁-C₂₀-Alkyl, C₃-C₁₄-Cycloalkyl, C₁-C₂₀-Alkoxy, Phenyl, Phenyloxy, oder Phenylthio, wobei Phenyl jeweils einoder mehrfach durch Halogen, C₁-C₂₀-Alkyl, C₃-C₁₄-Cycloalkyl und/oder C₁-C₂₀-Alkoxy substituiert sein kann, -NR⁵₂ oder -OR⁵ sein können, wobei R⁵ für Wasserstoff oder C₁-C₂₀-Alkyl steht, oder eines der Paare R¹/R² und R³/R⁴ jeweils in 6,7- oder 1,12-Position eine Brücke mit -O-, -S-, S=O, SO₂ oder -NR⁶ als Brückenatome bzw. -atomgruppen repräsentieren, wobei R⁶ für Wasserstoff, C₁-C₂₀-Alkyl oder C₃-C₁₄-Cycloalkyl steht.

Verfahren zur Herstellung von Perylen-3,4-dicarbonsäureanhydriden sind bekannt. So wird beispielsweise in Mol.Cryst.Liqu.Cryst., 158b, (1988), S. 337ff ein Verfahren beschrieben, in dem Perylen-3,4:9.10-tetracarbonsäurebisanhydrid durch Gasphasendecarboxylierung in Perylen-3,4-dicarbonsäureanhydrid überführt wird. Diese Vorgehensweise ist jedoch wegen der geringen Ausbeute (-5%) ohne praktische Bedeutung.

Ein weiteres mögliches Ausgangsprodukt zur Herstellung von Perylen-3,4-dicarbonsäureanhydriden stellt das technisch gut zugängliche Perylen-3,4-dicarboximid dar. Eine direkte Verseifung von Perylen-3,4-dicarboximiden ist mit Basen jedoch nicht möglich, da der Carboximidstickstoff deprotoniert wird und ein gegen Basen wie Alkalien inertes imidanion entsteht.

Eine Verseifung ist jedoch mit konzentrierter Schwefelsäure nach der in Bull. Chem. Soc. Jpn. 52 (1979) S. 1723ff beschriebenen Methode möglich. Ausgehend von Perylen-3,4-dicarboximid entsteht durch Erhitzen in konzentrierter Schwefelsäure auf eine Temperatur von etwa 250°C sulfoniertes Perylen-3,4-dicarbonsäureanhydrid. Die Autoren haben dieses Zwischenprodukt jedoch nicht isoliert, sondern mit Aminen weiter zu am Stickstoffatom substituierten Perylen-3,4-dicarboximid-sulfonsäuren umgesetzt und anschließend desulfoniert, um die entsprechenden am Stickstoffatom substituierten Perylen-3,4-dicarboximide zu erhalten. Nachteilig sind neben der umständlichen Reaktionsführung unter drastischen Bedingungen (konz. H₂SO₄ bei 250°C), die nur mäßigen Ausbeuten an Perylen-3,4-dicarboximiden.

Ein weiteres Verfahren ist aus DE-A 4,338,784 und EP-A 0 657 436 bekannt, nach dem ausgehend von Perylen-3,4:9,10-tetracarbonsäurebisanhydrid in einem ersten Schritt das N-(2,5-di-*tert*-butyl-phenyl)-perylen-3,4-dicarboximid hergestellt wird. Nach dessen Reinigung wird es durch Behandlung mit Alkali in das Perylen-3,4-dicarbonsäureanhydrid übergeführt. Nachteilig an diesem Verfahren ist, daß das N-(2,5-di-*tert*-butylphenyl)-perylen-3,4-dicarboximid chromatographisch gereinigt werden muß und daß das Endprodukt lediglich in einer Gesamtausbeute von 37%, bezogen auf Perylen-3,4:9,10-tetracarbonsäurebisanhydrid, entsteht. EP-A 0 657 436 beschreibt zudem die Herstellung von N-Alkyl- und N-Aralkylperylen-3,4-dicarboximiden aus Perylen-3,4-dicarboximid.

In der WO 96/22331 werden Perylen-3,4-dicarbonsäureimide sowie ein Verfahren zu deren Herstellung durch Umsetzung einer Perylen-3,4:9,10-tetracarbonsäure mit einem primären Amin in Gegenwart einer tertiären stickstoffbasischen Verbindung als Lösungsmittel und eines Übergangsmetalls als Katalysator beschrieben.

Aufgabe der vorliegenden Anmeldung war es daher, ein verbessertes Verfahren zur Herstellung von Perylen-3,4-dicarbonsäureanhydriden zur Verfügung zu stellen, das die oben genannten Nachteile nicht aufweist. Insbesondere sollte ausgehend von Perylen-3,4-dicarboximid und/oder seinen Derivaten in höheren Ausbeuten und in technischer Hinsicht einfacherer Verfahrensweise Perylen-3,4-dicarbonsäureanhydride hergestellt werden können.

Demgemäß wurde das erfindungsgemässe Verfahren zur Herstellung von Perylen-3,4-dicarbonsäureanhydriden der allgemeinen Formel I 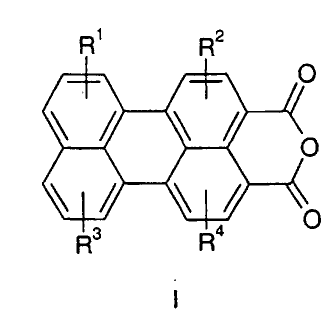 worin R¹, R², R³ und R⁴ unabhängig voneinander Wasserstoff, Halogen, C₁-C₂₀-Alkyl, C₃-C₁₄-Cycloalkyl, C₁-C₂₀-Alkoxy, Phenyl, Phenyloxy, oder Phenylthio, wobei Phenyl jeweils einoder mehrfach durch Halogen, C₁-C₂₀-Alkyl, C₃-C₁₄-Cycloalkyl und/oder C₁-C₂₀-Alkoxy substituiert sein kann, -NR⁵₂ oder -OR⁵ sein können, wobei R⁵ für Wasserstoff oder C₁-C₂₀-Alkyl steht, oder eines der Paare R¹/R² und R³/R⁴ jeweils in 6,7- oder 1,12-Position eine Brücke mit -O-, -S-, S=O, SO₂, oder -NR⁶ als Brückenatome bzw. -atomgruppen repräsentieren, wobei R⁶ für Wasserstoff, C₁-C₂₀-Alkyl oder C₃-C₁₄-Cycloalkyl steht, gefunden, indem man ein Perylen-3,4-dicarboximid der Formel II 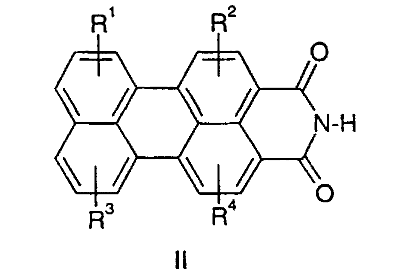
(a) in einem ersten Schritt mit einer Base behandelt, und
(b) in einem zweiten Schritt das entstandene Anion mit einem Alkylierungsmittel R⁷-X, wobei X für Halogen und R⁷ für unsubstituiertes oder mit Phenyl substituiertes C₁-C₂₀-Alkyl steht, zu dem entsprechenden Carboximid umsetzt, und
(c) in einem dritten Schritt zunächst das alkylierte Carboximid mit einer Base behandelt und nach der Basenbehandlung das Perylen-3,4-dicarbonsäureanhydrid I durch Ansäuern des Reaktionsgemisches erhält.

Die Behandlung des Carboximids II mit einer Base im ersten Schritt (a) nimmt man gewöhnlich bei einer Temperatur im Bereich von 0 bis 250°C, vorzugsweise bei Raumtemperatur, und gewünschtenfalls in Gegenwart eines Lösungsmittels, vor.

Als Base kann man üblicherweise Alkoholate oder Hydroxide von Alkalimetallen und Erdalkalimetallen einsetzen. Dabei sollte die Base zweckmässig so gewählt werden, dass sich das Wasserstoffatom, das sich am Imidstickstoffatom befindet, auch entfernen lässt. Man erhält dann ein Carboximid-Anion. Beispielsweise kann man als Alkoholate Alkalimetallsalze von C₁-C₄-Alkanolen wie Natriummethylat, Kaliummethylat, Natriumethylat oder Kaliumethylat verwenden. Als Alkalimetall- oder Erdalkalimetallhydroxide eignen sich beispielsweise Natriumhydroxid und Kaliumhydroxid. Besonders bevorzugt verwendet man Natriummethylat oder Kaliumhydroxid.

Das Molverhältnis von Dicarboximid II zu Base wählt man in der Regel im Bereich von 1:1 bis 0,01:1, bevorzugt von 0,33:1 bis 0,5 :1.

Als Lösungsmittel eignen sich bei Verwendung von Hydroxiden wie Kaliumhydroxid aprotische polare Lösungsmittel wie Dimethylsulfoxid ("DMSO") oder N-Methylpyrrolidon ("NMP"), bei Verwendung von Alkoholaten die entsprechenden Alkohole, also bei Verwendung von Natriummethylat bevorzugt Methanol usw. Die Menge des Lösungsmittels ist nach bisherigen Beobachtungen unkritisch und kann beispielsweise im Bereich von 100:1 bis 0,1:1 (Lösungsmittel zu Dicarboximid II, Gewichtsangaben) gewählt werden.

Vorzugsweise destilliert man das Lösungsmittel nach Beendigung der Reaktion ab. Ein Verbleib des Lösungsmittels im Reaktionsgemisch ist jedoch auch möglich.

Die Reaktionsdauer hängt üblicherweise im wesentlichen von der gewählten Reaktionstemperatur ab. In der Regel wählt man sie im Bereich von 0,5 bis 10 h.

Das als Ausgangsmaterial eingesetzte Dicarboximid II (R¹ bis R⁴ = Wasserstoff) ist beispielsweise aus der DE-C 486,491 durch Umsetzung des aus Perylen-3,4:9,10-tetracarbonsäurebisanhydrid erhältlichen Perylen-3,4-dicarbonsäureanhydrid-9,10-dicarboximids mit Kalilauge grosstechnisch erhältlich. Die substituierten Dicarboximide II können in Analogie zu dem in der DE-A 4,338,784 beschriebenen Verfahren hergestellt werden. So sind z.B. Nitro-Derivate durch Nitrierung mit Distickstofftetroxid in Dichlormethan oder Kupfernitrat in Acetanhydrid erhältlich. Durch die Reduktion der Nitroverbindungen kann man die entsprechenden Amino-Verbindungen synthetisieren, die wiederum weiter derivatisiert werden können. Bromierte Derivate können beispielsweise analog zu DE-A 4,338,784 durch direkte Bromierung hergestellt werden; hieraus sind in der Regel die entsprechenden Alkoxi- und Phenoxi-Derivate durch nukleophile Substitution zugänglich. Alkyl-Derivate können in Analogie zu Leonhard Feiler, Dissertation 1995, Universität München durch direkte Alkylierung mittels Alkyl-Lithium-Verbindungen erhalten werden.

Im zweiten Reaktionsschritt (b) wird erfindungsgemäss das Anion des Perylen-3,4-dicarboximids II mit dem Alkylierungsmittel, R⁷-X, umgesetzt.

X steht für Halogen wie Chlor, Brom oder lod, vorzugsweise für Brom oder lod, und R⁷ für unsubstituiertes oder mit Phenyl substituiertes C₁-C₂₀-Alkyl.

Als C₁-C₂₀-Alkyl setzt man beispielsweise ein Methyl, Ethyl, n-Propyl, Isopropyl, n-Butyl, sec.-Butyl, i-Butyl, tert.-Butyl, n-Amyl, tert.-Amyl, Hexyl, Heptyl, Octyl, 2-Ethylhexyl, Nonyl, Decyl, Dodecyl, Tetradecyl, Hexadecyl, Octadecyl, Nonadecyl oder Eicosyl ein, bevorzugt C₁-C₆-Alkyl wie Methyl, Ethyl, n-Propyl, Isopropyl, n-Butyl, sec.-Butyl, i-Butyl, tert.-Butyl, n-Amyl, tert.-Amyl, Hexyl.

Für den Fall, dass man mit der Phenylgruppe substituiertes Alkyl einsetzt, wählt man bevorzugt mit Phenyl einfach substituiertes C₁-C₄-Alkyl wie Benzyl, Phenylethyl, Phenylpropyl oder Phenylbutyl, besonders bevorzugt Benzyl.

Besonders bevorzugte Alkylierungsmittel sind Dimethylsulfat, C₁-C₆-Alkylbromide oder -iodide wie Methylbromid, Methyliodid, Ethylbromid, Ethyliodid, sowie Benzylchlorid.

Das Molverhältnis von Dicarboximid II zu Alkylierungsmittel wählt man in der Regel im Bereich von 1:1 bis 1:10, bevorzugt von 1:1 bis 1:4.

Gewünschtenfalls kann man die Alkylierungsreaktion in einem Lösungsmittel, bevorzugt in einem inerten, aprotisch polaren Lösungsmittel wie N-Methylpyrrolidon oder Dimethylsulfoxid durchführen. Die Menge an Lösungsmittel ist in der Regel unkritisch und liegt im allgemeinen im Bereich von 250:1 bis 0,1:1 (Masse Lösungsmittel zu Masse ursprünglich eingesetztes Dicarboximid II).

Die Reaktionstemperatur wählt man im allgemeinen im Bereich von 0 bis 150, bevorzugt von 20 bis 130°C. Insbesondere bei nicht eliminierenden Alkylierungsmitteln wie Benzylchlorid oder Dimethylsulfat wählt man bevorzugt einen höheren Temperaturbereich, während bei höheren Alkylierungsmitteln wie Octylbromid und bei niedrig siedenden Alkylierungsmitteln wie Methyliodid sowie Ethylbromid eher eine niedrige Reaktionstemperatur gewählt wird.

Die Reaktionsdauer hängt üblicherweise im wesentlichen von der gewählten Reaktionstemperatur und der Reaktivität der Reaktanden ab. In der Regel wählt man sie im Bereich von 0,3 bis 18 h.

In einer bevorzugten Ausführungsform destilliert man nach Beendigung der Alkylierung das Lösungsmittel zusammen mit nicht reagiertem Alkylierungsmittel ab. Das abgetrennte und gewünschtenfalls gereinigte Lösungsmittel lässt sich wiederverwenden.

Im dritten Reaktionsschritt (c) behandelt man zunächst erfindungsgemäss das im zweiten Schritt (b) gebildete N-Alkyl-perylen-3,4-dicarboximid mit einer Base und erhält danach das gewünschte Perylen-3,4-dicarbonsäureanhydrid I durch Ansäuern des Reaktionsgemisches.

Als Base im Schritt (c) verwendet man bevorzugt ein Alkalimetall- oder Erdalkalimetallhydroxid in einem primären, sekundären oder tertiären Alkohol mit maximal acht Kohlenstoffatomen.

Belspiele für Alkalimetall- oder Erdalkalimetallhydroxide sind Lithiumhydroxid, Natriumhydroxid, Kaliumhydroxid, Magnesiumhydroxid und Calciumhydroxid, besonders bevorzugt ist Kaliumhydroxid.

Das Molverhältnis von N-Alkyl-Dicarboximid zu Base wählt man üblicherweise im Bereich von 1:2 bis 1:25, vorzugsweise von 1:15 bis 1:20.

Beispielhaft seien folgende Alkohole als Lösungsmittel aufgeführt: primäre C₁-C₈-Alkanole wie Methanol, Ethanol, n-Propanol, n-Butanol, i-Butanol, n-Pentanol, n-Hexanol, n-Heptanol und n-Octanol; sekundäre C₃-C₈-Alkanole wie i-Propanol, sek.-Butanol, i-Pentanol, i-Hexanol, i-Heptanol und i-Octanol; tertiäre C₄-C₈-Alkanole wie tert.-Butanol, tert.-Amylalkohol, tert.-Hexanol, tert.-Heptanol und tert.-Octanol.

Die Menge an Lösungsmittel wählt man üblicherweise im Bereich von 25 bis 250, vorzugsweise von 70 bis 100 ml Lösungsmittel pro Gramm N-Alkyl-Dicarboximid.

Üblicherweise destilliert man das Lösungsmittel bei Reaktionsende ab. Man kann es gegebenenfalls nach Reinigung wiederverwenden.
Die Reaktionstemperatur wählt man im allgemeinen im Bereich von 20 bis 250, bevorzugt von 30 bis 100°C.

Die Reaktionsdauer hängt üblicherweise von der gewählten Reaktionstemperatur und der Reaktivität der Reaktanden ab. In der Regel beträgt sie von 0,5 bis 10 h.

In einer besonders bevorzugten Ausführungsform führt man die Verseifung mit Kaliumhydroxid in tert.-Butanol durch.

Das so erhaltene Perylen-3,4-dicarbonsäure-di-Salz wird erfindungsgemäss durch Ansäuern in das entsprechende Perylen-3,4-dicarbonsäureanhydrid überführt.

Zum Ansäuern kann man alle für eine Neutralisation geeigneten gängigen Säuren wie Mineralsäuren oder organische Säuren verwenden. Beispielhaft seien genannt: Schwefelsäure, Salzsäure, Salpetersäure, Phosphorsäure und Essigsäure.

Die Menge der Säure richtet sich in der Regel nach deren Konzentration und Säurestärke und wird üblicherweise so gewählt, dass das Reaktionsgemisch am Ende der Neutralisation einen pH-Wert im Bereich von 6,5 bis 7,5 aufweist.

Im allgemeinen isoliert man nach der Neutralisation das gewünschte Anhydrid I durch übliche Maßnahmen wie Filtration oder Entfernen des Lösungsmittels durch Destillation. Bevorzugt reinigt man das so erhaltene Rohprodukt durch Waschen mit Wasser, gewünschtenfalls Wasser mit einer Temperatur im Bereich von 40 bis 98°C ("heisses Wasser") und nachfolgendes Trocknen, vorzugsweise in einer Atmosphäre unter vermindertem Druck (beispielsweise erzeugt durch eine Wasserstrahlpumpe oder eine andere übliche Vakuumpumpe).

In einer bevorzugten Ausführungsform behandelt man das erhaltene Perylen-3,4-anhydrid I mit einer schwachen Base in wässrigem Milieu, wobei in der Regel das entsprechende Perylen-3,4-dicarbonsäure-di-Salz entsteht und in Lösung geht. Die nicht löslichen Bestandteile wie Perylen-3,4-dicarboximid und/oder N-Alkyl-perylen-3,4-dicarboximide kann man durch allgemein übliche Maßnahmen wie Filtration oder Zentrifugation abtrennen und gewünschtenfalls als Ausgangsmaterial einsetzen.

Das in Lösung befindliche Di-Salz säuert man dann nach Abtrennung der unlöslichen Bestandteile vorzugsweise mit einer mittelstarken oder starken Säure an, beispielsweise mit einer der bereits weiter oben genannten Säuren, und erhält nach Abtrennung mittels beispielsweise Filtration und anschliessender Trocknung, üblicherweise die erfindungsgemässen Perylen-3,4-dicarbonsäureanhydride in einer guten Reinheit.

Als schwache Basen kommen hierbei Alkalimetallcarbonate wie Natriumcarbonat und Kaliumcarbonat, besonders Kaliumcarbonat, in Frage. Bevorzugt erhitzt man das Rohprodukt in einer wässrigen Alkalimetallcarbonatlösung zum Sieden bis das gewünschte Produkt in Lösung ist und wäscht anschliessend nach der Abtrennung, bevorzugt Filtration, den Filterrückstand mit 40 bis 98°C heissem Wasser, bis das ablaufende Filtrat farblos ist.

Für chemische Umsetzungen ist das so erhaltene Perylen-3,4-dicarbonsäure-anhydrid in der Regel von ausreichender Reinheit. Höhere Reinheiten kann man z.B. durch extraktive Umkristallisation mit einem aromatischen Kohlenwasserstoff wie Toluol erreichen.

Die durch das erfindungsgemässen Verfahren hergestellten Perylenderivate I eignen sich zur Verwendung als Farbmittel, insbesondere als Pigmente und Farbstoffe, nach in der Regel jeweils an sich bekannten Methoden, bevorzugt
(a) zur Masse-Färbung von Polymeren, wobei man als Polymere Polyvinylchlorid, Celluloseacetat, Polycarbonaten, Polyamiden, Polyurethanen, Polyimiden, Polybenzimidazolen, Melaminharzen, Silikonen, Polyestern, Polyethern, Polystyrol, Polymethylmethacrylat, Polyethylen, Polypropylen, Polyvinylacetat, Polyacrylnitril, Polybutadien, Polychlorbutadien oder Polyisopren bzw. die Copolymeren der genannten Monomeren einsetzen kann;
(b) als Küpenfarbstoffe oder Beizenfarbstoffe, z.B. zur Färbung von Naturstoffen sowie insbesondere von Papier, Holz, Stroh, Leder, Felle oder natürlichen Fasermaterialien wie Baumwolle, Wolle, Seide, Jute, Sisal, Hanf, Flachs oder Tierhaare (z.B. Roßhaar) und deren Umwandlungsprodukte wie die Viskosefaser, Nitratseide oder Kupferrayon (Reyon), wobei bevorzugte Salze zum Beizen Aluminium-, Chrom- und Eisensalze sind;
(c) bei der Herstellung von Farben, Lacken, insbesondere Automobillacken, Anstrichstoffen, Papierfarben, Druckfarben, Tinten, insbesondere zum Einsatz in Tintenstrahldruckern, bevorzugt in homogener Lösung als fluoreszierende Tinte, und für Mal- und Schreib-Zwecke, sowie in der Elektrophotographie z.B. für Trockenkopiersysteme (Xerox-Verfahren) und Laserdrucker;
(d) für Sicherheitsmarkierungs-Zwecke wie für Schecks, Scheckkarten (Kreditkaten), Geldscheine, Coupons, Dokumente, Ausweispapiere und dergleichen, bei denen ein besonderer, unverkennbarer Farbeindruck erzielt werden soll;
(e) als Zusatz zu Farbmitteln wie Pigmenten und Farbstoffen, bei denen eine bestimmte Farbnuance erzielt werden soll, bevorzugt sind besonders leuchtende Farbtöne;
(f) zum Markieren von Gegenständen zum maschinellen Erkennen dieser Gegenstände über die Fluoreszenz, bevorzugt ist die maschinelle Erkennung von Gegenständen zum Sortieren, z.B. auch für das Recycling von Kunststoffen, wobei alphanumerische Aufdrucke oder Barcodes vorzugsweise eingesetzt werden;
(g) zur Frequenzumsetzung von Licht, z.B. um aus kurzwelligem Licht längerwelliges, sichtbares Licht zu machen oder zur Frequenzverdopplung und Frequenzverdreifachung von Laserlicht in der nichtlinearen Optik;
(h) zur Herstellung von passiven Anzeigeelementen für vielerlei Anzeige-, Hinweis- und Markierungszwecke, z.B. passive Anzeigeelemente, Hinweis- und Verkehrszeichen, wie Ampeln;
(i) als Ausgangsmaterial für supraleitende organische Materialien (über π-π-Wechselwirkungen, nach Dotierung mit z.B. lod erhält man üblicherweise eine intermedläre Ladungsdelokalisierung);
(j) zur Feststoff-Fluoreszenz-Markierung;
(k) für dekorative und künstlerische Zwecke;
(l) zu Tracer-Zwecken, z.B. in der Biochemie, Medizin, Technik und Naturwissenschaft, wobei die erfindungsgemässen Farbmittel kovalent mit Substraten oder über Nebenvalenzen wie Wasserstoffbrückenbindungen oder hydrophobe Wechselwirkungen (Adsorption) verknüpft sein können;
(m) als Fluoreszenzfarbstoffe in hochempfindlichen Nachweisverfahren (siehe C. Aubert, J. Fünfschilling, 1. Zschokke-Gränacher und H. Langhals, Z. Analyt. Chem. 1985, 320, 361), insbesondere als Fluoreszenzfarbstoffe in Szintillatoren;
(n) als Farbstoffe oder Fluoreszenzfarbstoffe in optischen Lichtsammelsystemen, in Fluoreszenz-Solarkollektoren (siehe H. Langhals, Nachr. Chem. Tech. Lab. 1980, 28, 716), in Fluoreszenz-aktivierten Displays (siehe W. Greubel und G. Baur, Elektronik 1977, 26, 6), in Kaltlichtquellen zur lichtinduzierten Polymerisation zur Darstellung von Kunststoffen, zur Materialprüfung, z.B. bei der Herstellung von Halbleiterschaltungen, zur Untersuchung von Mikrostrukturen von integrierten Halbleiterbauteilen, in Photoleitern, in fotografischen Verfahren, in Anzeige-, Beleuchtungs- oder Bildwandlersystemen, bei denen die Anregung durch Elektronen, lonen oder UV-Strahlung erfolgt, z.B. in Fluoreszenzanzeigen, Braunschen Röhren oder in Leuchtstoffröhren, als Teil einer integrierten Halbleiterschaltung, die Farbstoffe als solche oder in Verbindung mit anderen Halbleitern z.B. in Form einer Epitaxie, enthalten, in Chemilumineszenzsystemen, z.B. in Chemilumineszenz-Leuchtstäben, in Lumineszenzimmunassays oder anderen Lumineszenznachweisverfahren, als Signalfarben, bevorzugt zum optischen Hervorheben von Schriftzügen und Zeichnungen oder anderen graphischen Produkten, zum Kennzeichnen von Schildern und anderen Gegenständen, bei denen ein besonderer optischer Farbeindruck erreicht werden soll, in Farbstoff-Lasern, bevorzugt als Fluoreszenzfarbstoffe zur Erzeugung von Laserstrahlen, als optisches Speichermedium sowie als Q-Switch-Schalter;
(o) sowie als Rheologieverbesserer und als Farbstoffkomponente bei der Färbung von Metallen im ELOXAL-Verfahren.

Das erfindungsgemässe Verfahren hat gegenüber den zitierten Verfahren des Standes der Technik die Vorteile, dass es technisch einfacher ist ("Eintopfverfahren") und sehr gute Ausbeuten an Perylen-3,4-dicarboximiden liefert. Des weiteren werden aufwendige Reinigungsoperationen vermieden.

Ferner sind die nach dem erfindungsgemässen herstellbaren Perylenderivate I Farbstoffe oder Pigmente mit sehr hohen Lichtechtheiten sowie einer teilweise starken Festkörperfluoreszenz, die weitgehend das gesamte Lichtspektrum von gelb bis grün abdecken.

Die durch das erfindungsgemässe Verfahren hergestellten Anhydride I sind auch wegen ihrer Möglichkeit zur Derivatisierung durch die Umsetzung mit beispielsweise primären Aminen grosstechnisch von Interesse, da sich hierdurch die physikalischen Eigenschaften der erhaltenen Verbindungen in weitem Rahmen variiert lassen.

### Beispiele

Die Darstellung von Perylen-3,4-dicarboximid erfolgt analog zu der in der DE-C 486,491 beschriebenen Vorschrift, indem man 3,00g (7,67 mmol) Perylen-3,4-dicarboximid-9,10-dicarbonsäure-anhydrid zusammen mit 30 ml 12%ige Kalilauge suspendiert und die Mischung 18 h lang im Autoklaven auf 240 bis 250°C erhitzt. Nach dem Abkühlen neutralisiert man mit konz. Salzsäure. Der rotbraune Niederschlag wird abgesaugt, mehrmals mit dest. Wasser gewaschen und anschliessend im Trockenschrank bei 120 °C getrocknet. So erhaltenes Perylen-3,4-dicarboximid kann ohne weitere Reinigung für Umsetzungen verwendet werden. Ausbeute: 2,22 g ( 90 %).

Beispiel 1: Man suspendiert 1,60 g (5,2 mmol) Perylen-3,4-dicarboximid in 50 ml abs. Methanol, fügt 0.56 g (14.7 mmol) Natriummethylat dazu und rührt die Mischung unter Feuchtigkeitsausschluß bei Raumtemperatur 2 h. Das Methanol wird am Rotationsverdampfer abgezogen, dann fügt man 60 ml N-Methylpyrrolidon als Lösungsmittel und 2.28 g (16.0 mmol) Methyliodid dazu und rührt die Suspension 2 h lang bei Raumtemperatur. Nach Beendigung der Reaktion wird das N-Methylpyrrolidon und überschüssiges Methyliodid in einer Atmosphäre unter vermindertem Druck abdestilliert.

Der rotbraune Rückstand wird anschließend in 150 ml *tert*.-Butanol suspendiert, mit 6.0 g (107 mmol) KOH-Plätzchen versetzt und 3h lang gekocht. Hierbei kann man die Umfärbung der Mischung von rotbraun nach gelbbraun beobachten. Nach Beendigung der Reaktion destilliert man das *tert*-Butanol ab, suspendiert den Rückstand in Wasser und säuert langsam mit konz. Salzsäure an. Damit sich der entstehende braune Niederschlag zusammenballt, kocht man die Mischung einige Zeit. Der Niederschlag wird abgesaugt und mit heißem Wasser gewaschen. Der Rückstand wird mit 2 N Kaliumcarbonatlösung ausgekocht und dann so lange mit heißem Wasser gewaschen, bis das Filtrat farblos abläuft. Der Rückstand besteht aus nicht umgesetztem Perylen-3,4-dicarboximid und geringen Mengen N-Methyl-perylen-3,4-dicarboximid, und kann als Ausgangsprodukt wiederverwendet werden.
Aus dem Filtrat werden durch Ansäuern mit Salzsäure und anschließender Filtration 1.20 g (75 %) braunrotes Perylen-3,4-dicarbonsäureanhydrid erhalten.
IR (KBr): ν= 1780 cm⁻¹ (w), 1750 (m, C=O), 1725 (m, C=O), 1589 (s), 1568 (m), 1339 (m), 1280 (s), 1130 (m), 1020 (m), 1002 (m), 860 (w), 843 (m), 812 (s), 767 (s), 740 (m).

Beispiel 2: Man suspendiert 1,60 g (5,2 mmol) Perylen-3,4-dicarboximid in 50 ml abs. Methanol, fügt 0,56 g (14,7 mmol) Natriummethylat dazu und rührt die Mischung unter Feuchtigkeitsausschluß bei Raumtemperatur 2 h. Das Methanol wird am Rotationsverdampfer abgezogen, dann fügt man 60 ml N-Methylpyrrolidon als Lösungsmittel und 2,04 g (16,1 mmol) Benzylchlorid dazu und rührt die Suspension 12 lang bei 50°C. Nach Beendigung der Reaktion wird das N-Methylpyrrolidon und überschüssiges Benzylchlorid im Vakuum abdestilliert.

Der rotbraune Rückstand wird anschließend in 150 ml *tert*-Butanol suspendiert, mit 6.0 g (107 mmol) KOH-Plätzchen versetzt und 3h lang gekocht. Hierbei kann man die Umfärbung der Mischung von rotbraun nach gelbbraun beobachten. Nach Beendigung der Reaktion destilliert man das tert-Butanol ab, suspendiert den Rückstand in Wasser und säuert langsam mit konz. Salzsäure an. Damit sich der entstehende braune Niederschlag zusammenballt kocht man die Mischung einige Zeit. Der Niederschlag wird abgesaugt und mit heißem Wasser gewaschen. Der Rückstand wird mit 2 N Kaliumcarbonatlösung ausgekocht und dann so lange mit heißem Wasser gewaschen, bis das Filtrat farblos abläuft. Der Rückstand besteht aus nicht umgesetztem Perylen-3,4-dicarboximid und geringen Mengen N-Benzyl-perylen-3,4-dicarboximid, und kann als Ausgangsprodukt wiederverwendet werden.
Aus dem Filtrat werden durch Ansäuern mit Salzsäure und anschließender Filtration 1.12 g (70 %) braunrotes Perylen-3,4-dicarbonsäureanhydrid erhalten.
IR (KBr): ν = 1780 cm⁻¹ (w), 1750 (m, C=O), 1725 (m, C=O), 1589 (s), 1568 (m), 1339 (m), 1280 (s), 1130 (m), 1020 (m), 1002 (m), 860 (w), 843 (m), 812 (s), 767 (s), 740 (m).

Beispiel 3: Man suspendiert 0.40 g (1.25 mmol) Perylen-3,4-dicarboximid in 25 ml abs. Methanol, gibt 0.17 g (1.9 mmol) Natriummethylat dazu und rührt die Mischung ¹/₂ h lang bei Raumtemperatur unter Feuchtigkeitsausschluß. Anschließend wird das Lösungsmittel abdestilliert, 20 ml abs. N-Methylpyrrolidon und 0.54 g (2.81 mmol) 1-Bromoctan dazugegeben und 23 h bei Raumtemperatur gerührt. Nach Beendigung der Reaktion wird das N-Methylpyrrolidon und überschüssiges 1-Bromoctan im Vakuum abdestilliert.

Der rotbraune Rückstand wird anschließend in 60 ml *tert*-Butanol suspendiert, mit 1.5 g (22.8 mmol) KOH-Plätzchen versetzt und 3h lang gekocht. Hierbei kann man die Umfärbung der Mischung von rotbraun nach gelbbraun beobachten. Nach Beendigung der Reaktion destilliert man das tert-Butanol ab, suspendiert den Rückstand in Wasser und säuert langsam mit konz. Salzsäure an. Damit sich der entstehende braune Niederschlag zusammenballt kocht man die Mischung einige Zeit. Der Niederschlag wird abgesaugt und mit heißem Wasser gewaschen. Der Rückstand wird mit 2 N Kaliumcarbonatlösung ausgekocht und dann so lange mit heißem Wasser gewaschen, bis das Filtrat farblos abläuft. Der Rückstand besteht aus nicht umgesetztem Perylen-3,4-dicarboximid und geringen Mengen N-Octyl-perylen-3,4-dicarboximid, und kann als Ausgangsprodukt wiederverwendet werden.
Aus dem Filtrat werden durch Ansäuern mit Salzsäure und anschließender Filtration 260 mg (65 %) braunrotes Perylen-3,4-dicarbonsäureanhydrid erhalten. (Die Ausbeute läßt sich weiter verbessern, wenn man den Rückstand wieder als Ausgangsprodukt einsetzt.)
IR (KBr): ν = 1783 cm⁻¹ (w), 1753 (m, C=O), 1728 (m, C=O), 1592 (s), 1570 (m), 1501 (w), 1405 (w), 1372 (w), 1342 (m), 1285 (s), 1231 (w), 1132 (m), 1021 (m), 1000 (m), 860 (w), 840 (m), 815 (s), 770 (s), 740 (m).
C₂₂H₁₀O₃ (322.3) Ber. C 81.98 H 3.12 Gef. C 81.69 H 3.24.

Beispiel 4: Man suspendiert 0.40 g (1.3 mmol) Perylen-3,4-dicarboximid in 25 ml abs. Methanol, fügt 0.19 g (3.5 mmol) Natriummethylat dazu und rührt die Mischung unter Feuchtigkeitsausschluß bei Raumtemperatur 2 h. Das Methanol wird am Rotationsverdampfer abgezogen, dann fügt man 20 ml N-Methylpyrrolidon als Lösungsmittel und 1.13 g (4.03 mmol) 1-Bromtetradecan dazu und rührt die Suspension 24 h lang bei Raumtemperatur. Nach Beendigung der Reaktion wird das N-Methylpyrrolidon und überschüssiges 1-Bromtetradecan im Vakuum abdestilliert.
Der rotbraune Rückstand wird anschließend in 60 ml *tert*-Butanol suspendiert, mit 1.5 g (22.8 mmol) KOH-Plätzchen versetzt und 3h lang gekocht. Hierbei kann man die Umfärbung der Mischung von rotbraun nach gelbbraun beobachten. Nach Beendigung der Reaktion destilliert man das *tert*-Butanol ab, suspendiert den Rückstand in Wasser und säuert langsam mit konz. Salzsäure an. Damit sich der entstehende braune Niederschlag zusammenballt, kocht man die Mischung einige Zeit. Der Niederschlag wird abgesaugt und mit heißem Wasser gewaschen. Der Rückstand wird mit 2 N Kaliumcarbonatlösung ausgekocht und dann so lange mit heißem Wasser gewaschen, bis das Filtrat farblos abläuft. Der Rückstand besteht aus nicht umgesetztem Perylen-3,4-dicarboximid und geringen Mengen N-Tetradecyl-perylen-3,4-dicarboximid, und kann als Ausgangsprodukt wiederverwendet werden.
Aus dem Filtrat werden durch Ansäuem mit Salzsäure und anschließender Filtration 240 mg (60 %) braunrotes Perylen-3,4-dicarbonsäureanhydrid erhalten.
IR (KBr): ν= 1780 cm⁻¹ (w), 1750 (m, C=O), 1725 (m, C=O), 1589 (s), 1568 (m), 1339 (m), 1280 (s), 1130 (m), 1020 (m), 1002 (m), 860 (w), 843 (m), 812 (s), 767 (s), 740 (m).

Beispiel 5: Man suspendiert 1,29 g (4 mmol) Perylen-3,4-dicarboximid, 0,56 g (14,7 mmol) Natriummethylat in 50 ml Ethanol und rührt die Mischung 5 h lang bei Raumtemperatur. Das Lösungsmittel wird in einer Atmosphäre unter vermindertem Druck abgedampft, man gibt 50 ml N-Methylpyrrolidon und 1,01 ml Methyliodid zu und rührt 2h lang bei Raumtemperatur. Das Lösungsmittel wird in einer Atmosphäre unter vermindertem Druck abdestilliert, der Rückstand mit 100 ml Schwefelsäure (20%ig) gewaschen, abgesaugt und bei 130 °C in einer Atmosphäre unter vermindertem Druck getrocknet. Man erhält 1,17 g (87 %) rotes N-Methyl-perylen-3,4-dicarboximid.
IR (KBr): ν = 1692 cm⁻¹ (s), 1657 (s), 1592 (s), 1570 (s), 1361 (s), 1283 (m), 810 (s), 748 (m).
C₂₃H₁₃O₂N (335,36) Ber. C 82,37 H 3,91N 4,18 Gef. C 81,71 H 4,01 N 3,98

Das Produkt wird mit 6,0 g KOH-Plätzchen und 100 ml *tert.*-Butylalkohol versetzt und 5 h lang unter Rückfluss gekocht. Nach dem Abkühlen auf Raumtemperatur gibt man 100 ml Phosphorsäure (30 %) zu und erhitzt die Mischung 15 min. Der Niederschlag wird abgesaugt, mit Wasser gewaschen und anschliessend 30 min lang mit 200 ml 2 N Kaliumcarbonatlösung gekocht. Es wird heiss vom Ungelösten abgesaugt und solange mit heissem Wasser nachgewaschen, bis das Filtrat farblos abläuft. Das Filtrat wird in noch heissem Zustand mit 100 ml Essigsäure (50%) versetzt, der braune Niederschlag abgesaugt und das erhaltene braune Perylen-3,4-dicarbonsäureanhydrid in einer Atmosphäre unter vermindertem Druck bei 130 °C getrocknet.
Ausbeute: 0,73 g (56%) braunes Pulver
IR (KBr): ν = 1750 cm⁻¹ (s), 1726 (s), 1591 (s), 1569 (s), 1340 (s), 1283 (s), 1131 (m), 1021 (m), 810 (m), 742 (m)
C₂₂H₁₀O₃ (322,32) Ber.C 81,98 H 3,13 Gef. C 80,71 H 3,39

Beispiel 6: Man suspendiert 1,29 g (4 mmol) Perylen-3,4-dicarboximid, 1,35 g (12 mmol) Kalium-*tert*.-butylat in 100 ml Ethanol und kocht die Mischung 2h lang unter Rückfluss. Das Lösungsmittel wird in einer Atmosphäre unter vermindertem Druck abgedampft, man gibt 100 ml N-Methylpyrrolidon zu und erwärmt auf 95 °C. Anschliessend gibt man 1,82 ml (16 mmol) Benzylchlorid zu und erwärmt 30 min lang unter starkem Rühren auf 120 °C. Das Lösungsmittel wird in einer Atmosphäre unter vermindertem Druck abdestilliert, der Rückstand mit 6,0 g KOH-Plätzchen und 100 ml *tert.*-Amylalkohol versetzt und 3 h lang unter Rückfluss gekocht. Nach dem Abkühlen auf Raumtemperatur gibt man 100 ml Essigsäure (50 %) zu und erhitzt die Mischung eine Stunde um den Niederschlag besser filtrierbar zu machen. Der Niederschlag wird abgesaugt, mit Wasser gewaschen und anschliessend 30 min lang mit 100 ml 2 N Kaliumcarbonatlösung gekocht. Es wird heiss vom Ungelösten abgesaugt und solange mit heissem Wasser nachgewaschen, bis das Filtrat farblos abläuft. Das Filtrat wird in noch heissem Zustand mit 100 ml Essigsäure (50%) versetzt, der braune Niederschlag abgesaugt und das erhaltene braune Perylen-3,4-dicarbonsäureanhydrid in einer Atmosphäre unter vermindertem Druck bei 130 °C getrocknet.
Ausbeute: 0,47 g ( 37 %)
IR (KBr): ν = 1752 cm⁻¹ (s), 1725 (s), 1591 (s), 1569 (s), 1341 (s), 1283 (s), 1131 (m), 1020 (m), 810 (m), 741 (m)
C₂₂H₁₀O₃ (322,32), Ber. C 81,98 H 3,13; Gef. C 81,22 H 3,42

Beispiel 7: Man suspendiert 1,29 g (4 mmol) Perylen-3,4-dicarboximid, 1,35 g (12 mmol) Kalium-*tert.*-butylat in 100 ml Ethanol und kocht die Mischung 1h lang unter Rückfluss. Das Lösungsmittel wird in einer Atmosphäre unter vermindertem Druck abgedampft, man gibt 100 ml N-Methylpyrrolidon zu und erwärmt auf 95 °C. Anschliessend gibt man 1,82 ml (16 mmol) Benzylchlorid zu und erwärmt 50 min lang unter starkem Rühren auf 120 °C. Das Lösungsmittel wird in einer Atmosphäre unter vermindertem Druck abdestilliert, der Rückstand mit 5,1 g KOH-Plätzchen und 100 ml *tert.*-Butylalkohol versetzt und 2 h 30 min. lang unter Rückfluss gekocht. Nach dem Abkühlen auf Raumtemperatur gibt man 50 ml Schwefelsäure (30%) und 50 ml Wasser zu und erhitzt die Mischung eine Stunde um den Niederschlag besser filtrierbar zu machen. Der Niederschlag wird abgesaugt, mit Wasser gewaschen und anschliessend 1 h 30 min lang mit 100 ml 2 N Kaliumcarbonatlösung gekocht. Es wird heiss vom Ungelösten abgesaugt und solange mit heissem Wasser nachgewaschen, bis das Filtrat farblos abläuft. Das Filtrat wird in noch heissem Zustand mit 100 ml Essigsäure (50%) versetzt, der braune Niederschlag abgesaugt und das erhaltene braunes Perylen-3,4-dicarbonsäureanhydrid in einer Atmosphäre unter vermindertem Druck bei 130°C getrocknet.
Ausbeute: 0,31 g (24 %)
IR (KBr): ν = 1750 cm⁻¹ (s), 1722 (s), 1591 (s), 1569 (s), 1341 (s), 1283 (s), 1131 (m), 1019 (m), 810 (m), 741 (m)

Beispiel 8: Man suspendiert 1,29 g (4 mmol) Perylen-3,4-dicarboximid und 1,80 g (16 mmol) Kalium-*tert.*-butylat in 100 ml *tert.*-Amylalkohol und kocht die Mischung 30 min lang unter Rückfluss. Anschliessend gibt man 2,30 ml (20 mmol) Benzylchlorid zu und kocht 7 h unter Rückfluss. Das Lösungsmittel wird in einer Atmosphäre unter vermindertem Druck abdestilliert, der Rückstand mit 100 ml Phosphorsäure (30 %) versetzt und abgesaugt. Der Rückstand wird mit 8,0 g KOH-Plätzchen und 100 ml *tert.*-Amylalkohol versetzt und 4 h 30 min. lang unter Rückfluss gekocht. Nach dem Abkühlen auf Raumtemperatur gibt man 100 ml Schwefelsäure (30%) zu. Das Lösungsmittel wird in einer Atmosphäre unter vermindertem Druck abgedampft, der Niederschlag wird abgesaugt, mit Wasser gewaschen und anschliessend 45 min lang mit 100 ml 2 N Kaliumcarbonatlösung gekocht. Man saugt vom Ungelösten ab und wäscht solange mit heissem Wasser nach, bis das Filtrat farblos abläuft. Dazu gibt man 50 ml Schwefelsäure (30 %), saugt den ausgefallenen Niederschlag ab und trocknet in einer Atmosphäre unter vermindertem Druck bei 130 °C.
Ausbeute: 0,14 g (11%)
IR (KBr): ν = 1755 cm⁻¹ (s), 1725 (s), 1591 (s), 1569 (s), 1341 (s), 1284 (s), 1141 (m), 1284 (m), 1019 (m), 810 (m), 741 (m)

Beispiel 9: Man suspendiert 1,29 g (4 mmol) Perylen-3,4-dicarboximid und 0,90 g (8 mmol) Kalium-*tert.*-butylat in 100 ml Glycolmonomethylether und rührt die Mischung 1h lang bei 70 °C. Das Lösungsmittel wird in einer Atmosphäre unter vermindertem Druck abgedampft, man gibt 120 ml N-Methylpyrrolidon und 0,95 ml (10 mmol) Dimethylsulfat zu und rührt 4 h 30 min bei 70 °C. Das Lösungsmittel wird in einer Atmosphäre unter vermindertem Druck abdestilliert, der Rückstand mit 8,0 g KOH-Plätzchen und 100 ml *tert*.-Butylalkohol versetzt und 14 h lang unter Rückfluss gekocht. Nach dem Abkühlen auf Raumtemperatur gibt man 100 ml Phosphorsäure (30%) zu. Das Lösungsmittel wird in einer Atmosphäre unter vermindertem Druck abgedampft, der Niederschlag wird abgesaugt, mit Wasser gewaschen und anschliessend 30 min lang mit 200 ml 2 N Kaliumcarbonatlösung gekocht. Man saugt vom Ungelösten ab und gibt 150 ml Phosphorsäure (30 %) zu der Lösung dazu. Die Mischung wird 15 min. lang erhitzt, dann saugt man den ausgefallenen Niederschlag ab und trocknet in einer Atmosphäre unter vermindertem Druck bei 130°C.
Ausbeute: 0,29 g (22 %)
IR (KBr): ν = 1750 cm⁻¹ (s), 1724 (s), 1591 (s), 1569 (s), 1341 (s), 1283 (s), 1131 (m), 1019 (m), 997 (m), 810 (m), 741 (m)
C₂₂H₁₀O₃ (322,32) Ber. C 81,98 H 3,13 Gef. C 80,34 H 3,38

## Patentansprüche

1. Verfahren zur Herstellung von Perylen-3,4-dicarbonsäureanhydriden der allgemeinen Formel I 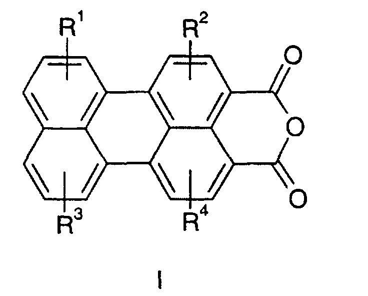 worin R¹, R², R³ und R⁴ unabhängig voneinander Wasserstoff, Halogen, C₁-C₂₀-Alkyl, C₃-C₁₄-Cycloalkyl, C₁-C₂₀-Alkoxy, Phenyl, Phenyloxy, oder Phenylthio, wobei Phenyl jeweils einoder mehrfach durch Halogen, C₁-C₂₀-Alkyl, C₃-C₁₄-Cycloalkyl und/oder C₁-C₂₀-Alkoxy substituiert sein kann, -NR⁵₂ oder -OR⁵ sein können, wobei R⁵ für Wasserstoff oder C₁-C₂₀-Alkyl steht, oder eines der Paare R¹/R² und R³/R⁴ jeweils in 6,7- oder 1,12-Position eine Brücke mit -O-, -S-, S=O, SO₂, oder -NR⁶ als Brückenatome bzw. -atomgruppen repräsentieren, wobei R⁶ für Wasserstoff, C₁-C₂₀-Alkyl oder C₃-C₁₄-Cycloalkyl steht,
**dadurch gekennzeichnet, dass** man ein Perylen-3,4-dicarboximid der Formel II 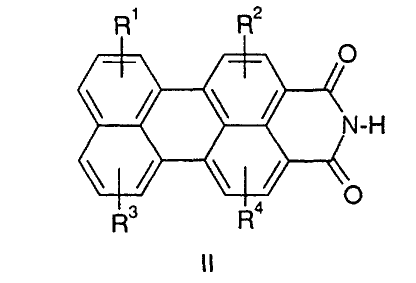
(a) in einem ersten Schritt mit einer Base behandelt, und
(b) in einem zweiten Schritt das entstandene Anion mit einem Alkylierungsmittel, R⁷-X, wobei X für Halogen und R⁷ für unsubstituiertes oder mit Phenyl substituiertes C₁-C₂₀-Alkyl steht, zu dem entsprechenden Carboximid umsetzt, und
(c) in einem dritten Schritt zunächst das alkylierte Carboximid mit einer Base behandelt und nach der Basenbehandlung das Perylen-3,4-dicarbonsäureanhydrid I durch Ansäuern des Reaktionsgemisches erhält.

2. Verfahren nach Anspruch 1, **dadurch gekennzeichnet, dass** man als Base im ersten Schritt (a) ein Alkalimetallsalz eines C₁-C₄-Alkanols oder ein Alkalimetall- oder Erdalkalimetallhydroxid verwendet.

3. Verfahren nach den Ansprüchen 1 oder 2, **dadurch gekennzeichnet, dass** man als Alkylierungsmittel Dimethylsulfat, C₁-C₆-Alkylbromide, -iodide oder Benzylchlorid einsetzt.

4. Verfahren nach den Ansprüchen 1 bis 3, **dadurch gekennzeichnet, dass** man die Reaktionen der Schritte (a) bis (c) jeweils in einem Lösungsmittel durchführt.

## Claims

1. A process for the preparation of perylene-3,4-dicarboxylic acid anhydrides of the general formula I 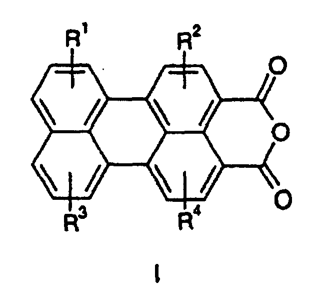 wherein R¹, R², R³ and R⁴ can each be independently of one another hydrogen, halogen, C₁-C₂₀alkyl, C₃-C₁₄-cycloalkyl, C₁-C₂₀alkoxy, phenyl, phenyloxy or phenylthio, where phenyl can in each case be mono- or polysubstituted by halogen, C₁-C₂₀alkyl, C₃-C₁₄cycloalkyl and/or C₁-C₂₀alkoxy; -NR⁵₂ or -OR⁵, where R⁵ is hydrogen or C₁-C₂₀alkyl, or one of the pairs R¹/R² and R³/R⁴ each in 6,7- or 1,12-position is a bridge having the bridge atoms or bridge atom groups -O-, -S-, S=O, SO₂ or -NR⁶, where R⁶ is hydrogen, C₁-C₂₀alkyl or C₃-C₁₄cycloalkyl, which comprises treating a perylene-3,4-dicarboximide of formula II 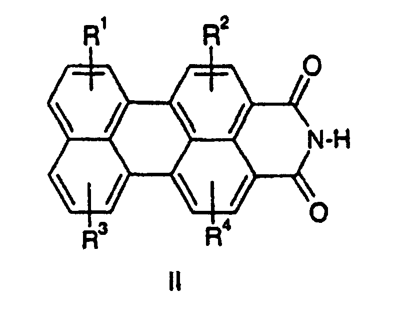
(a) in a first step with a base, and
(b) in a second step, reacting the resultant anion with an alkylation agent R⁷-X, X being halogen and R⁷ being unsubstituted or phenyl-substituted C₁-C₂₀alkyl, to the corresponding carboximide, and
(c) in a third step, first treating the alkylated carboximide with a base and, after the treatment with a base, obtaining the perylene-3,4-dicarboxylic acid anhydride I by acidifying the reaction mixture.

2. A process according to claim 1, wherein the base used in the first step (a) is an alkali metal salt of a C₁-C₄alkanol or an alkali metal hydroxide or alkaline earth metal hydroxide.

3. A process according to either claim 1 or claim 2, wherein the alkylation agent used is dimethyl sulfate, C₁-C₆alkyl bromides, C₁-C₆alkyl iodides or benzyl chloride.

4. A process according to any one of claims 1 to 3, wherein each of the reactions of steps (a) to (c) is carried out in a solvent.

## Revendications

1. Procédé pour la préparation d'anhydrides d'acide pérylène-3,4-dicarboxylique de formule générale I 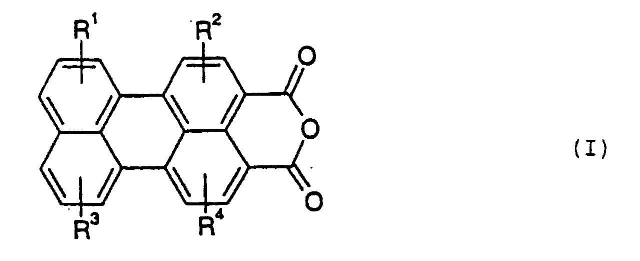 où R¹, R², R³ et R⁴ peuvent représenter, indépendamment les uns des autres, des atomes d'hydrogène, des atomes d'halogènes, des groupes alkyle en C₁-C₂₀, cycloalkyle en C₃-C₁₄, alkoxy en C₁-C₂₀, phényle, phényloxy ou phénylthio, phényle pouvant être chaque fois substitué une ou plusieurs fois par des substituants halogène, alkyle en C₁-C₂₀, cycloalkyle en C₃-C₁₄ et/ou alkoxy en C₁-C₂₀, représentent des groupes -NR⁵₂ ou -OR⁵, où R⁵ représente un atome d'hydrogène ou un groupe alkyle en C₁-C₂₀, ou une des paires R¹/R² et R³/R⁴ représente chaque fois en. positions 6,7 ou 1,12 un pont avec des groupes -O-, -S-, S=O, SO₂ ou -NR⁶ en tant qu'atomes de pont ou groupes d'atomes de pont, R⁶ représentant un atome d'hydrogène, des groupes alkyle en C₁-C₂₀ ou cycloalkyle en C₃-C₁₄, en traitant un pérylène-3,4-dicarboximide de formule II 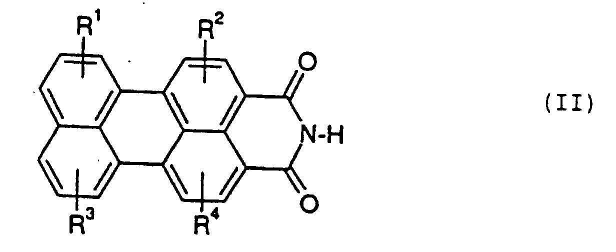
(a) dans une première étape par une base, et
(b) dans une deuxième étape, l'anion formé réagit sur un agent d'alkylation R⁷-X, où X représente un atome d'halogène et R⁷ représente un groupe alkyle en C₁-C₂₀ non substitué ou substitué par un substituant phényle, pour obtenir le carboximide correspondant, et
(c) dans une troisième étape, on traite d'abord le carboximide alkylé par une base et après le traitement par la base, on obtient l'anhydride d'acide pérylène-3,4-dicarboxylique I par acidification du mélange réactionnel.

2. Procédé selon la revendication 1, **caractérisé en ce qu'**on utilise comme base dans la première étape (a) un sel de métal alcalin d'un alcanol en C₁-C₄ ou d'un hydroxyde de métaux alcalins ou alcalino-terreux.

3. Procédé selon les revendications 1 ou 2, **caractérisé en ce qu'**on utilise comme agent d'alkylation le sulfate de diméthyle, des bromures, des iodures d'alkyles en C₁-C₆ ou le chlorure de benzyle.

4. Procédé selon les revendications 1 à 3, **caractérisé en ce qu'**on met en oeuvre les réactions des étapes (a) à (c) chaque fois dans un solvant.
